# EUROPEAN PATENT SPECIFICATION

(11) **EP 3 295 885 B1**
(45) Date of publication and mention of the grant of the patent: **30.11.2022**
(21) Application number: 16792177.4
(22) Date of filing: 10.05.2016
(51) Int. Cl.: A61B 18/18

(54) **CORRUGATED RADIOFREQUENCY ABLATION CATHETER HAVING WALL-ATTACHING ADJUSTMENT WIRES AND APPARATUS THEREOF**
GEWELLTER HOCHFREQUENZ-ABLATIONSKATHETER MIT WANDFIXIERUNGSANPASSUNGSDRÄHTEN UND VORRICHTUNG DAFÜR
CATHÉTER D'ABLATION PAR RADIOFRÉQUENCES ONDULÉ COMPORTANT DES CÂBLES DE RÉGLAGE ASSURANT LA FIXATION À LA PAROI ET APPAREIL ASSOCIÉ

(30) Priority: 13.05.2015 CN 201510244254; 12.08.2015 CN 201520605029 U; 12.08.2015 CN 201510492572
(43) Date of publication of application: 21.03.2018
(73) Proprietor: Shanghai Golden Leaf Med Tec Co., Ltd., Shanghai 200231 (CN)
(72) Inventor: DONG, Yonghua, Shanghai 200231 (CN); SHEN, Meijun, Shanghai 200231 (CN)
(74) Representative: Keil & Schaafhausen Patentanwälte PartGmbB
(86) International application number: PCT/CN2016/081621
(87) International publication number: WO 2016/180327

(56) References cited:
- WO-A1-2014/166436
- CN-A- 101 309 651
- CN-A- 102 488 552
- CN-A- 104 127 233
- CN-A- 105 078 571
- CN-U- 205 019 161
- US-A1- 2001 020 174
- US-A1- 2002 004 644

## Description

### Technical Field

The present invention relates to a corrugated radio frequency ablation catheter having a wall-attaching adjustment wire, also relates to a radio frequency ablation apparatus comprising the radiofrequency ablation catheter, and belongs to the technical field of interventional medical instruments.

### Related Art

In a radiofrequency ablation system, a radiofrequency ablation catheter is a key device used for human blood vessel intervention and radiofrequency energy release. Radiofrequency electrodes are installed on a frame at the front extremity of the radiofrequency ablation catheter, the frame is used for bearing the radiofrequency electrodes, and the frame expands to be attached to the wall before radiofrequency starts and retracts after radiofrequency ends. Since radiofrequency ablation surgery is conducted through direct human blood vessel intervention, the expanding and retracting size of the frame needs to be matched with the diameters of human blood vessels.

The diameters of human blood vessels vary with ablation portions. Besides, the diameters of human blood vessels vary from person to person. For example, the renal artery diameters of different persons range from 2 mm to 12 mm, showing a great difference. In the prior art, the expanding and retracting size of the electrode end of the radiofrequency ablation catheter is generally fixed and can not adapt to different diameters of human blood vessels, thus being small in coverage over human blood vessels with different diameters. Therefore, when radiofrequency ablation surgery is conducted on different patients, radiofrequency ablation catheters of different specifications and models are usually required for ablation. Even so, the problem that radiofrequency electrodes can not be attached to the wall at the same time still exists during certain surgery, and the surgical effect is influenced.

Radio frequency ablation catheters can be of various structures based on the shape of electrodes and the shape of an electrode frame, such as a balloon type, a puncture needle type, a spiral type and a lobe structure. The adaptability of all existing radio frequency ablation catheters to blood vessels with different diameters is limited.

### SUMMARY

The primary technical problem to be solved by the present invention is to provide a corrugated radio frequency ablation catheter having a wall-attaching adjustment wire.

Another technical problem to be solved by the present invention is to provide a radio frequency ablation apparatus comprising the radio frequency ablation catheter.

In order to achieve the above-mentioned purposes, the present invention adopts the following technical scheme:
according to a first aspect of an embodiment of the present invention, the corrugated radio frequency ablation catheter having the wall-attaching adjustment wire is provided, the corrugated radiofrequency ablation catheter is provided with a strip-shaped connecting catheter, an electrode frame is provided at the front extremity of the connecting catheter, and a control handle is provided at the rear extremity of the connecting catheter;
wherein the electrode frame is a corrugated electrode frame consisting of one or more corrugations, where one or more electrodes are distributed on the corrugations;
the rear section of the wall-attaching adjustment wire is slidably provided within one lumen of the connecting catheter and is connected at the rear extremity onto a control element provided on the control handle or connected onto a control element provided outside of the control handle; and the front section of the wall-attaching adjustment wire protrudes to the outside of the electrode frame and either runs through one or more holes provided on the corrugations or runs around the multiple corrugations, and then the front extremity returns to the interior of the electrode frame to be fixed.

Preferably, after returning to the interior of the electrode frame, the front extremity of the wall-attaching adjustment wire run through lumens in the electrode frame and the connecting catheter, returns to the rear extremity of the connecting catheter, and is fixed to the control handle or the control element.

Or, preferably, the front extremity of the wall-attaching adjustment wire is fixed to the front extremity of the electrode frame after returning to the interior of the electrode frame.

Or, preferably, after protruding out of the front extremity of the electrode frame, the front extremity of the wall-attaching adjustment wire is fixed to the front extremity of the electrode frame or limited outside the front extremity of the electrode frame.

Or, preferably, the corrugated radio frequency ablation catheter further comprises a supporting wire provided within a certain lumen of the connecting catheter and the electrode frame, and the front extremity of the wall-attaching adjustment wire is fixed to the supporting wire; or the wall-attaching adjustment wire is a filament obtained through outward branching of the supporting wire.

Preferably, the portion, inside the electrode frame, of the supporting wire is shaped into a corrugation shape, so as to form a corrugation shaping section.

Or, preferably, the corrugated radio frequency ablation catheter further comprises a shaping wire provided within a certain lumen of the electrode frame, and the front extremity of the wall-attaching adjustment wire is fixed to the shaping wire; or the wall-attaching adjustment wire is a filament obtained through outward branching of the shaping wire.

Or, preferably, the wall-attaching adjustment wire is composed of two or more filaments, the multiple filaments are used for adjusting one corrugation or one section of corrugations on the electrode frame respectively, one section of corrugations comprises two or more corrugations, the front extremity of each filament is fixed to one end of the corresponding corrugation/corrugation section, and the other extremity of each filament runs around the corresponding corrugation/corrugation section, runs through lumens in the electrode frame and the connecting catheter, and is then fixed to the corresponding control element provided on the control handle or arranged externally.

Preferably, the multiple sections of corrugations controlled by the multiple filaments respectively overlap.

Preferably, the wall-attaching adjustment wire is eccentrically provided on the electrode frame.

Preferably, the electrodes are arranged at the crests/troughs of the corrugations.

According to a second aspect of the embodiment of the present invention, a radio frequency ablation apparatus is provided, and the radio frequency ablation apparatus comprises the radio frequency ablation catheter and a radio frequency ablation main unit connected with the radiofrequency ablation catheter.

The corrugated radiofrequency ablation catheter having the wall-attaching adjustment wire is novel in structure and can be well adapted to target lumens with different diameters. In target lumens with different diameters, by pulling the wall-attaching adjustment wire, the electrodes provided on the corrugations can be well attached to the wall. Preferably, by designing the distribution positions of different corrugations in the corrugated radiofrequency ablation catheter, the multiple electrodes located on the corrugations can be distributed into an approximate circle around a target lumen when being attached to the wall. Furthermore, the wall-attaching adjustment wire can be of a multi-filament structure, different corrugation sections of the radiofrequency ablation catheter can be controlled by independently controlling each filament, and the difficulty of diameter adjustment of the corrugated radiofrequency ablation catheter can be simplified.

WO2014/166436 discloses a multi-electrode ablation catheter having a helical support frame. US 2002004644 also discloses a helical support structure according to the prior art.

### BRIEF DESCRIPTION OF THE DRAWINGS

Fig. 1A and Fig. 1B are a three-dimensional structure diagram and a side view of a corrugated radio frequency ablation catheter in a first embodiment respectively;
Fig. 2 is a cross section view of an electrode frame of the corrugated radio frequency ablation catheter shown in Fig. 1A;
Fig. 3 is an E-E section view of the electrode frame shown in Fig. 2;
Fig. 3A is an enlarged view of an F portion shown in Fig. 3;
Fig. 4 is a view of another arrangement mode of a wall-attaching adjustment wire in the first embodiment;
Fig. 5 is a structure diagram of a control handle of the corrugated radio frequency ablation catheter shown in Fig. 1A in the first embodiment;
Fig. 6A is a use state view of the corrugated radiofrequency ablation catheter entering a target lumen with a small diameter in the first embodiment;
Fig. 6B is a side view corresponding to Fig. 6A;
Fig. 7A is a use state view of the corrugated radio frequency ablation catheter entering a target lumen with a large diameter in the first embodiment;
Fig. 7B is a side view corresponding to Fig. 7A;
Fig. 8A and Fig. 8B are a front view and a side view of a corrugated radio frequency ablation catheter in a second embodiment respectively;
Fig. 9A and Fig. 9B are a three-dimensional structure diagram and a side view of a corrugated radio frequency ablation catheter in a third embodiment respectively;
Fig. 10A and Fig. 10B are a three-dimensional structure diagram and a side view of a corrugated radiofrequency ablation catheter in a fourth embodiment respectively;
Fig. 11 is a three-dimensional structure diagram of a corrugated radiofrequency ablation catheter in a fifth embodiment;
Fig. 12 is a first exemplary structure of a wall-attaching adjustment wire in the corrugated radiofrequency ablation catheter shown in Fig. 11;
Fig. 13 is a second exemplary structure of the wall-attaching adjustment wire in the corrugated radiofrequency ablation catheter shown in Fig. 11;
Fig. 14 is a third exemplary structure of the wall-attaching adjustment wire in the corrugated radiofrequency ablation catheter shown in Fig. 11;
Fig. 15A is a first use state view of a control handle of the corrugated radiofrequency ablation catheter in the fifth embodiment;
Fig. 15B is a second use state view of the control handle of the corrugated radiofrequency ablation catheter in the fifth embodiment;
Fig. 16 is a three-dimensional structure diagram of a corrugated radiofrequency ablation catheter in a sixth embodiment;
Fig. 17 is a three-dimensional structure diagram of a second corrugated radiofrequency ablation catheter in the sixth embodiment;
Fig. 18 is a three-dimensional structure diagram of a corrugated radiofrequency ablation catheter in a seventh embodiment;
Fig. 19 is a first exemplary structure of a wall-attaching adjustment wire in the corrugated radio frequency ablation catheter shown in Fig. 18;
Fig. 20 is a second exemplary structure of the wall-attaching adjustment wire in the corrugated radio frequency ablation catheter shown in Fig. 18;
Fig. 21 is a third exemplary structure of the wall-attaching adjustment wire in the corrugated radio frequency ablation catheter shown in Fig. 18;
Fig. 22 is a three-dimensional structure diagram of a corrugated radiofrequency ablation catheter in an eighth embodiment;
Fig. 23 is a structure diagram of a wall-attaching adjustment wire in the corrugated radiofrequency ablation catheter shown in Fig. 22;
Fig. 24 is a three-dimensional structure diagram of a corrugated radio frequency ablation catheter in a ninth embodiment.

### DETAILED DESCRIPTION

The technical content of the present invention is further described in detail with reference to accompanying drawings and specific embodiments.

### First Embodiment

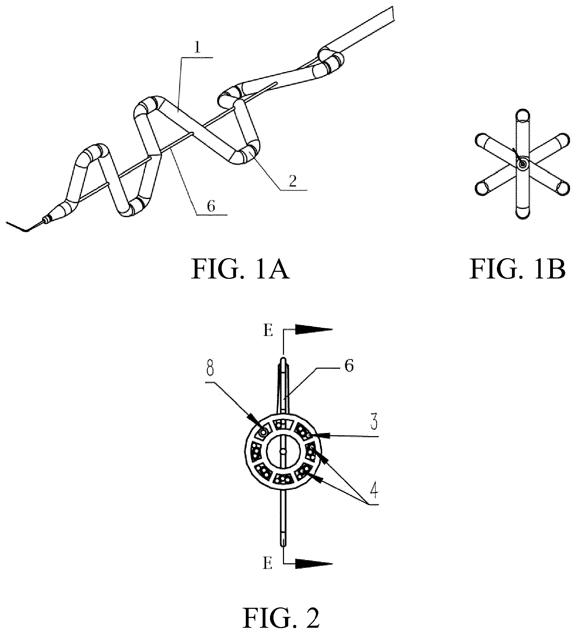

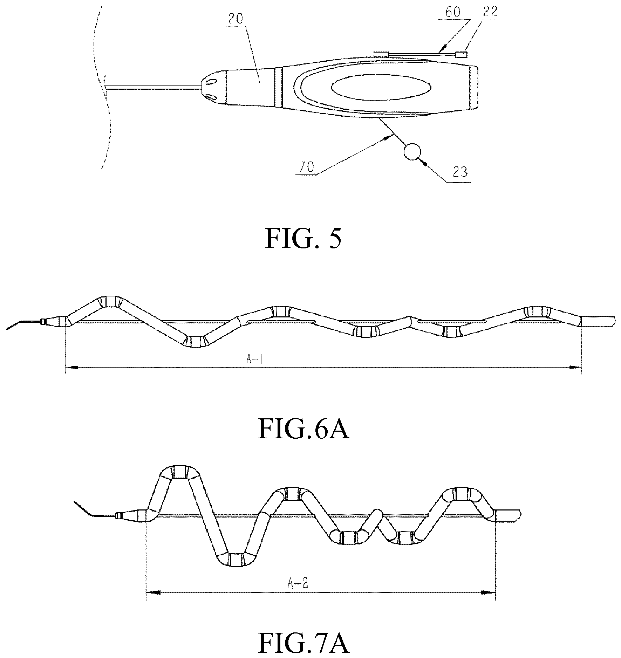

It can be learnt from Fig 1A to Fig. 7B that a corrugated radiofrequency ablation catheter provided by the present invention comprises a strip-shaped connecting catheter, a corrugated electrode frame is provided at the front extremity of the connecting catheter (see Fig. 1A), and a control handle 20 is provided at the rear extremity of the connecting catheter (see Fig. 5). During actual manufacturing, the electrode frame can be manufactured integral with the connecting catheter, the connecting frame is the part configured to be corrugated at the front extremity of the connecting catheter, and the electrode frame can also be independently manufactured and then connected with the connecting catheter into a whole.

As shown in Fig. 1A and Fig. 1B, the corrugated electrode frame comprises an outer tube 1 and one or more electrodes 2 provided on the outer tube 1. The outer tube 1 is configured to be a corrugated shape consisting of one or more corrugations; each corrugation can be in the shape of a fold line composed of several straight line segments, such as a triangular wave; each corrugation can also be composed of several curve segments, such as a sine wave or an arc wave; and each corrugation can also be composed of curves and straight lines, such as a trapezoidal wave with a corner. Besides, the corrugations can also be in other corrugated shapes. Furthermore, in the same electrode frame, the multiple corrugations can be in the same shape and same size, and can also be in different shapes and different sizes. Detailed explanation will be given with reference to specific embodiments. Among the multiple corrugations, part of the corrugations are located in different planes, and part of the corrugations are located in the same plane. In the present embodiment, every two corrugations are located in the same plane, so that the profile projections of the multiple corrugations are in a radial shape as shown in Fig. 1B. The multiple electrodes 2 can be distributed on the corrugations respectively, and preferably, the electrodes 2 are provided at the crests or troughs of the corrugations. The electrodes 2 can be block type electrodes or annular electrodes embedded in the outer periphery of the outer tube 1, the external surfaces of the electrodes 2 can be flush with the external surface of the outer tube 1 or slightly higher than the external surface of the outer tube 1, and the external surfaces of the electrodes 2 can also be lower than the external surface of the outer tube 1.

It can be learnt from the side view as shown in Fig. 1B that in the present embodiment, the profile projections of the corrugations in the corrugated electrode frame are distributed in a crossing mode, and the multiple electrodes 2 are arranged at the crest positions respectively. When the corrugations cross each other at the same angle, the profile projections, on the electrode frame, of the multiple electrodes 2 can be evenly distributed in the circumferential direction, in other words, the profile projections are distributed on the outer periphery of a target lumen into an approximate circle. Of course, when the crossing angles of the corrugations are different, the profile projections, on the electrode frame, of the multiple electrodes 2 can be distributed unevenly in the circumferential direction. Besides, when the electrode frame is long, the multiple corrugations, in the length direction, of the electrode frame can be repeated regularly or randomly, so that the profile projections, on the electrode frame, of the multiple electrodes 2 can overlap.

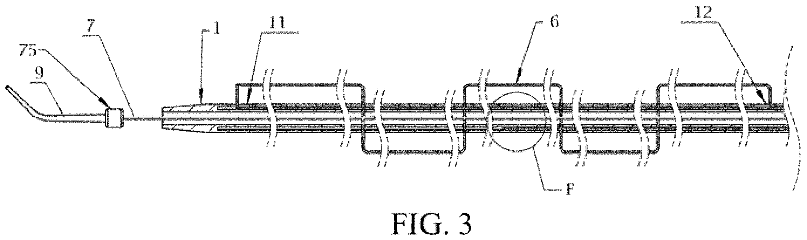

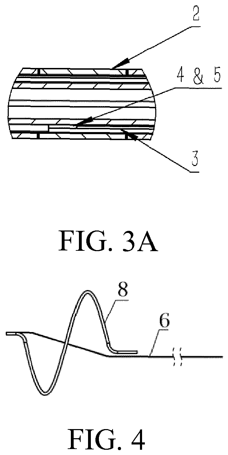

It can be learnt from internal section views as shown in Fig. 2, Fig. 3 and Fig. 3A that the outer tube 1 of the electrode frame can be a single-lumen tube or a multi-lumen tube, and the outer tube 1 can be made of a polymer material or a metal material, such as stainless steel or memory alloy. The outer tube 1 can be machined from straight tubing and bars, and can also be made into a corrugated special-shaped tube with the segment A. As shown in Fig. 2, when the outer tube 1 is a multi-lumen tube, multiple lumens are provided inside the outer tube 1 of the electrode frame besides a central lumen, and a set of radio frequency cables 3 and thermocouple wires 4 is arranged in part of the lumens; the head ends of each set of radio frequency cables 3 and thermocouple wires 4 are arranged in a single electrode 2, wherein the head ends of the radio frequency cables 3 are tightly fixed to the electrode 2 through welding, conductive adhesive gluing or other techniques, the head ends of the two thermocouple wires 4 are welded and coated with thermocouple wire head end insulating layers 5, and then insulated from the radio frequency cables 3 and the electrode 2.

As shown in Fig. 2, a shaping wire 8 is further arranged in one lumen of the outer tube 1, and the shaping wire 8 is fixed in a deformation section of the electrode frame to be used for supporting the corrugated shape of the electrode frame. Of course, the electrode frame can also be directly shaped into a corrugated shape, so that the shaping wire 8 can be omitted, for example, when the outer tube is made of memory alloy or a polymer material, the outer tube can be directly shaped, so that the shaping wire 8 can be omitted.

As shown in Fig. 3, a supporting wire 7 is arranged in the central lumen inside the connecting catheter and the electrode frame, the supporting wire 7 can be movably arranged in the central lumen and can also be fixedly arranged in the central lumen, or the supporting wire 7 can be movably or fixedly arranged in other lumens of the connecting catheter and the electrode frame. A camera head 75 can be arranged at the head end of the supporting wire 7 to be used for real-time imaging of the interior of a target lumen. Meanwhile, a soft guide wire 9 can also be arranged at the front extremity of the supporting wire 7, the soft guide wire 9 can be a straight-head soft guide wire, and can also be a bent-head soft guide wire as shown in the figure, in this way, the radio frequency ablation catheter can directly enter blood vessels without a guide catheter/sheath, and surgical procedures are simplified.

It can be learnt from Fig. 2 to Fig. 5 that a lumen used for accommodating the wall-attaching adjustment wire 6 is further provided in the outer tube and the connecting catheter, and the rear section of the wall-attaching adjustment wire 6 is slidably provided within one lumen of the connecting catheter and is connected at the rear extremity 60 onto a control element 22 provided on a control handle 20 (see Fig. 5). The wall-attaching adjustment wire 6 can slide back and forth in the lumen of the connecting catheter. The lumen for accommodating the wall-attaching adjustment wire 6 can be the central lumen or one of the multiple eccentric lumens distributed on the periphery of the central lumen. As shown in Fig. 1A, the front section of the wall-attaching adjustment wire 6 runs through a hole 12 near the rear extremity of the electrode frame and multiple holes provided on different corrugations, and finally the front extremity of the wall-attaching adjustment wire 6 runs through a hole 11 near the front extremity of the electrode frame and returns to the interior of the electrode frame to be fixed. The wall-attaching adjustment wire 6 can slide in the holes provided on different corrugations.

The front extremity of the wall-attaching adjustment wire 6 can be fixed at different positions, and the front extremity of the wall-attaching adjustment wire 6 can be fixed to the front extremity of the electrode frame, to the front extremity of the supporting wire 7, or to the shaping wire 8, or the front extremity of the wall-attaching adjustment wire 6 runs through corresponding lumens in the electrode frame 2 and the connecting catheter to be fixed to the control element 22 or a housing of the control handle 20 together with the rear extremity 60 of the wall-attaching adjustment wire 6.

Specifically, as shown in Fig. 3, after running through the hole 11 near the front extremity of the electrode frame and returning to the interior of the electrode frame 2, the front extremity of the wall-attaching adjustment wire 6 runs through the lumens in the electrode frame and the connecting catheter and returns to the rear extremity of the connecting catheter together with the rear extremity of the wall-attaching adjustment wire 6, and is fixed to the housing of the control handle 20 or the control element 22. In other words, the front extremity and the rear extremity of the wall-attaching adjustment wire 6 can be fixed to the same control element 22 as shown in Fig. 5, or either the front extremity or the rear extremity of the wall-attaching adjustment wire 6 is fixed to the housing of the control handle 20, and the other one is fixed to the control element 22. By pulling the control element 22, the wall-attaching adjustment wire 6 is driven to move backwards, and the diameter of the electrode frame can be changed.

Of course, the front extremity of the wall-attaching adjustment wire 6 can also be simply fixed to the front extremity of the electrode frame, or fixed to the front extremity of the supporting wire 7 or a certain portion, located in the electrode frame, of the supporting wire 7, or fixed to a certain position on the shaping wire 8, or the front extremity of the wall-attaching adjustment wire 6 is fixed in the lumen of the electrode frame, as long as the front extremity of the wall-attaching adjustment wire 6 is fixed, in this way, when the wall-attaching adjustment wire 6 is pulled back, contraction distortion of the electrode frame can be caused under the action of the wall-attaching adjustment wire 6, the diameters of the corrugations of the electrode frame are increased, and the axial distance between the multiple corrugations becomes smaller. When the front extremity of the wall-attaching adjustment wire 6 is fixed to the supporting wire 7 or the shaping wire 8, the wall-attaching adjustment wire 6 and the supporting wire 7/shaping wire 8 can be made of the same material, and in this case, the wall-attaching adjustment wire 6 can be interpreted as a filament obtained through backward branching of the supporting wire 7/shaping wire 8.

For example, as shown in Fig. 4, the front extremity of the wall-attaching adjustment wire 6 and the front extremity of the shaping wire 8 are fixed together, in this case, the shaping wire 8 and the wall-attaching adjustment wire 6 can be made of the same kind of filament, the wall-attaching adjustment wire 6 and the shaping wire 8 are two filament branches obtained through backward branching of the front extremity of the filament respectively, wherein the branch corresponding to the shaping wire 8 is fixed in a certain lumen of the electrode frame, and the rear section of the branch corresponding to the wall-attaching adjustment wire 6 can slide in the lumen of the electrode frame and/or a body of the catheter. When the wall-attaching adjustment wire 6 and the shaping wire 8 are made of different materials (for example, the shaping wire 8 is made of tubing and the wall-attaching adjustment wire 6 is made of a filament), the front extremity/front section of the wall-attaching adjustment wire 6 and the shaping wire 8 can be assembled together through welding, riveting, bonding or other techniques.

Besides, it can be seen from Fig. 5 that in the above-mentioned structure, a control element 23 is further arranged outside the control handle 20, and the tail end 70 of the supporting wire 7 also enters the control handle 20 after protruding to the outside of the connecting catheter, and is fixed to the externally arranged control element 23 after passing through the control handle 20. Of course, the control element 22 connected with the wall-attaching adjustment wire 6 can also be provided outside the control handle 20 in an externally arranged way, and the front extremity and/or the rear extremity of the wall-attaching adjustment wire 6 passes through the control handle 20 and then is connected to the externally arranged control element 22. Similarly, the control element 23 can also be arranged on the control handle 20, and the supporting wire 7 penetrates into the control handle 20 and is then directly connected with the control element 23. When the supporting wire 7 is fixedly arranged in the connecting catheter and the electrode frame, the control element 23 used for controlling the supporting wire 7 can be omitted.

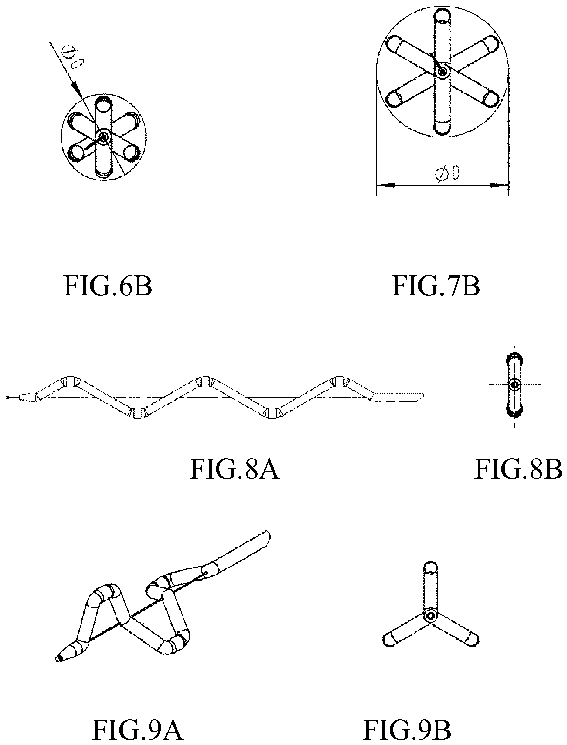

Fig. 6A to Fig. 7B show the use state views of the corrugated radiofrequency ablation catheter entering target lumens with different diameters. Suppose the corrugated electrode frame as shown in Fig. 1A has an initial diameter of ΦB and a corrugation section length of A. By loosening the wall-attaching adjustment wire 6, the wall-attaching adjustment wire 6 becomes loose, at the moment, the length of a corrugation section at the front extremity of the catheter can be increased by means of a sheath, so that the corrugation section is approximately straight and can enter a target lumen. As shown in Fig. 6A, when the corrugated electrode frame enters a thin blood vessel through the sheath (suppose that the diameter of the target lumen ΦC is smaller than the initial diameter of the corrugations ΦB), the corrugations of the electrode frame automatically expand to have a diameter close to the diameter of the target lumen ΦC (see Fig. 6B), the multiple electrodes 2 make contact with the wall of the catheter under the natural expansion of the electrode frame, at the moment, the length of the corrugation section of the electrode frame is increased to be (A-1), and the wall-attaching state of the electrodes 2 can be improved by tensioning the wall-attaching adjustment wire 6. As shown in Fig. 7A, when the corrugated electrode frame runs through the sheath and enters a thick blood vessel (suppose that the diameter of the target lumen is larger than or equal to the initial diameter of the corrugations ΦB), the electrodes 2 can not be well attached to the wall after the electrode frame expands naturally, at the moment, by pulling back the wall-attaching adjustment wire 6, the diameters of the corrugations of the electrode frame can be increased to be equal to or slightly larger than the diameter of the target lumen ΦD (see Fig. 7B), and the multiple electrodes 2 make close contact with the wall of the catheter under the action of the wall-attaching adjustment wire 6. At the moment, the length of the corrugation section of the electrode frame is shortened to be (A-2), and the axial distance between the multiple electrodes distributed on the electrode frame becomes smaller. After radio frequency ends, by loosening the wall-attaching adjustment wire 6, the electrode frame becomes loose, then the electrode frame is made to enter the sheath by moving the sheath forward or moving the catheter backward, so that the radio frequency ablation catheter can be rotated or moved in the target lumen, or moved out of the target lumen.

### Second to fifth embodiments

In the second embodiment as shown in Fig. 8A and Fig. 8B, the corrugated electrode frame is composed of multiple triangular waves, and the multiple corrugations are located in the same plane. The multiple electrodes are located at the crests and troughs of the triangular waves respectively, and due to the fact that the profile projections of the multiple triangular waves overlap, the profile projections of the multiple electrodes overlap too. After ablation is finished once, the catheter can be rotated by a certain angle to conduct ablation on the same position of the target lumen again.

In the third embodiment as shown in Fig. 9A and Fig. 9B, the corrugated electrode frame is composed of multiple arc waves, but the multiple corrugations are located in different planes. The multiple electrodes are located at the crests (also called troughs) of the arc waves respectively, so that the profile projections of the multiple electrodes can be distributed in the circumferential direction of the target lumen. At the moment, after ablation is finished once, the catheter can be directly moved to conduct ablation on other positions of the target lumen, and the operation of rotating the catheter at the same position of the target lumen is omitted.

In the second embodiment and the third embodiment, after running through the hole near the rear extremity of the electrode frame, the front section of the wall-attaching adjustment wire 6 runs through the holes provided on different corrugations, and finally the front extremity of the wall-attaching adjustment wire 6 runs through the hole near the front extremity of the electrode frame and returns to the interior of the electrode frame to be fixed.

Due to the fact that in the third embodiment, the multiple arc waves are located in different planes and the profile projections of the multiple electrodes are distributed in the circumferential direction of the target lumen, compared with the second embodiment, the requirement of radio frequency ablation surgery for the arrangement direction of the electrode frame in the target lumen is low in the third embodiment, and therefore operation is easy. However, the structure of the second embodiment can enter the target lumen more easily than the structure of the third embodiment.

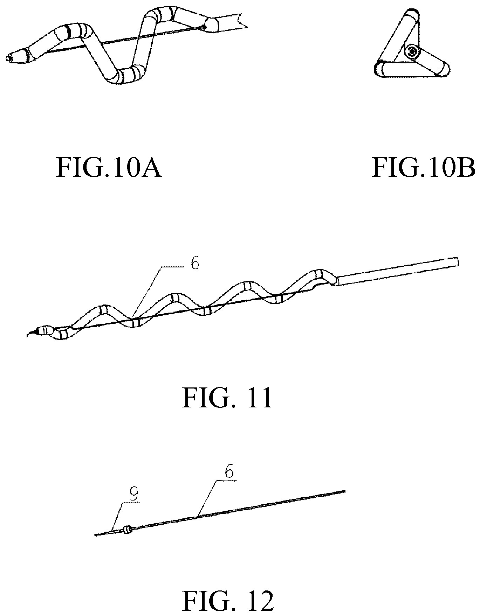

In the fourth embodiment as shown in Fig. 10A and Fig. 10B, the multiple corrugations of the corrugated electrode frame are all located in different planes, moreover, the multiple corrugations are distributed into an approximate spiral shape, the multiple electrodes are located at the crests (also called troughs) of the corrugations respectively, and therefore the multiple electrodes can be distributed in the circumferential direction of the target lumen. In the present embodiment, the multiple corrugations can be distributed into one or more circles of spirals, and moreover, the wall-attached adjustment wire 6 can also run through the holes provided on the different corrugations.

In the fifth embodiment as shown in Fig. 11, the corrugated electrode frame is composed of multiple sine waves. Like the second embodiment, the multiple corrugations in the fifth embodiment are located in the same plane, and moreover, the multiple electrodes are located at the crests and troughs of the sine waves respectively. But different from the second embodiment, the front section of the wall-attached adjustment wire 6 is fixed at the front extremity after running around the multiple corrugations instead of running through the multiple corrugations.

It can be learnt from the above five embodiments that the multiple corrugations in the corrugated electrode frame can be in the shape of a triangular wave (see Fig. 8A) composed of several straight line segments, an arc wave (see Fig. 10A) or sine wave (see Fig. 11) composed of several arc segments, a trapezoidal wave composed of straight lines and curves, or any other corrugations not shown in the figures. The multiple corrugations can be distributed in the same plane, can also be distributed in different planes, and can even be distributed into an approximate spiral shape in an encircling mode, so that the electrodes can be distributed in the circumferential direction. Compared with the situation that the multiple corrugations are distributed in the same plane, when the multiple corrugations are distributed in different planes, the corrugated electrode frame can be attached to the wall in any direction in the target lumen during actual ablation surgery. In the above-mentioned embodiments as shown in the figures, on the same electrode frame, the multiple corrugations forming the corrugated shape are in the same shape. Of course, the multiple corrugations forming the corrugated shape can have different shapes and sizes, and the corrugations can be different in form, spacing, crest position, trough position and the like. When the corrugated electrode frame consists of corrugations in different sizes, the wall-attaching states of local electrodes can be adjusted by adjusting the sizes of the corrugations in a local area, and at the same time when the wall-attaching states are adjusted, and meanwhile, the forms of other areas may not be adjusted. Wall-attaching adjustment of the corrugated electrode frame consisting of different corrugations can be achieved by pulling different filament branches in the wall-adjustment adjustment wire 6 composed of multiple filaments. Please see the ninth embodiment for the structure of the wall-adjustment adjustment wire 6 composed of multiple filaments and the wall-attaching adjustment way.

Besides, in the radio frequency ablation catheter, the wall-adjustment adjustment wire 6 can be arranged in multiple ways, the front section of the wall-adjustment adjustment wire 6 can run through the holes in the outer tube provided with the multiple corrugations as in the first, second and third embodiments, and can also directly run around the corrugations and then enter the electrode frame to be fixed instead of passing through the outer tube with the multiple corrugations. Compared with the arrangement mode that the front section of the wall-adjustment adjustment wire 6 is entirely exposed from the electrode frame, by allowing the wall-adjustment adjustment wire 6 to run through the holes in different corrugations of the outer tube, the shape change of the electrode frame is controllable, and the wall-attaching effect is better.

### Fifth embodiment

Only the form of the electrode frame and the arrangement mode of the front section of the wall-adjustment adjustment wire 6 are briefly introduced above, now detailed explanation will be given on the specific structure of the wall-adjustment adjustment wire 6 in the radio frequency ablation catheter and the structure of the corresponding control handle 20 with reference to Fig. 11 to Fig. 15B and the fifth embodiment.

According to the radio frequency ablation catheter as shown in Fig. 11, the structure of the wall-adjustment adjustment wire 6 inside the radio frequency ablation catheter can be similar to the structure in the first embodiment, that is to say, the wall-adjustment adjustment wire 6 is a monofilament independent of the supporting wire 7 and the shaping wire 8; the wall-adjustment adjustment wire 6 also has the function of the supporting wire, or the front extremity of the wall-adjustment adjustment wire 6 can be fixed to the supporting wire 7 to serve as a branch of the supporting wire 7. Both the supporting wire 7 and the wall-adjustment adjustment wire 6 can be made of a filament or a thin tube.

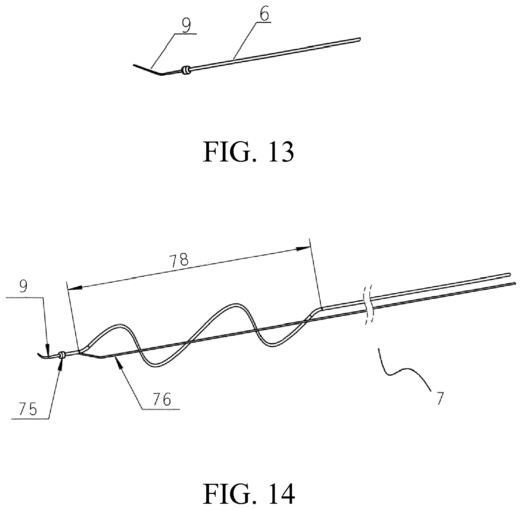

As shown in Fig. 12 and Fig. 13, when the wall-adjustment adjustment wire 6 has the function of the supporting wire, the rear section of the wall-adjustment adjustment wire 6 is slidably provided within a certain lumen of the connecting catheter and connected at the rear extremity onto the control handle 20; and after the front section of the wall-attaching adjustment wire 6 runs around the multiple corrugations or runs through the holes provided on the different corrugations, the front extremity of the wall-attaching adjustment wire 6 runs through the hole 11 near the front extremity of the electrode frame, returns to the interior of the electrode frame, protrudes out of the front extremity of the electrode frame and is fixed to the front extremity of the electrode frame or limited outside the front extremity of the electrode frame. A camera head and/or soft guide wire 9 can be provided at the front extremity of the wall-attaching adjustment wire 6. The soft guide wire 9 can be a straight-head soft guide wire as shown in Fig. 12, and can also be a bent-head soft guide wire as shown in Fig. 13. The bent-head soft guide wire can be composed of multiple arcs, straight lines or curves, and can have one or more elbows. When the soft guide wire is provided at the front extremity of the wall-attaching adjustment wire 6, the radio frequency ablation catheter can enter a blood vessel and reach a required position under the guidance of a sheathless pipe.

As shown in Fig. 14, when the front extremity of the wall-attaching adjustment wire 6 is fixed to the supporting wire 7, the wall-attaching adjustment wire 6 can be seen as a backward branch 76 of the supporting wire 7. In this case, one or two lumens exist inside the connecting catheter and the electrode frame to be used for accommodating two branches of the supporting wire 7. When no shaping wire 8 is independently arranged inside the electrode frame, the portion, corresponding to the electrode frame, of the front part of the supporting wire 7 can be configured to be a corrugation shaping section 78 through pre-shaping, the branch, corresponding to the corrugation shaping section 78, of the supporting wire 7 is fixed to the interior of the corresponding lumen, and the rear extremity can be directly fixed in the connecting catheter and can also be fixed in the control handle, so that the electrode frame can remain in a corrugated shape when no external force is applied; the branch 76, corresponding to the wall-attaching adjustment wire, of the supporting wire 7 can be slidably arranged in the lumen, and the tail end can be fixed to the control element provided on the control handle 20 or to the control element arranged externally. When the supporting wire 7 does not have the function of the shaping wire 8, the supporting wire 7 and the wall-attaching adjustment wire 6 can be slidably arranged in the same lumen or in two different lumens, the rear extremities of the supporting wire 7 and the wall-attaching adjustment wire 6 are fixed to the corresponding control element provided on the control handle 20 or to the corresponding control element arranged externally after protruding to the outside of the connecting catheter.

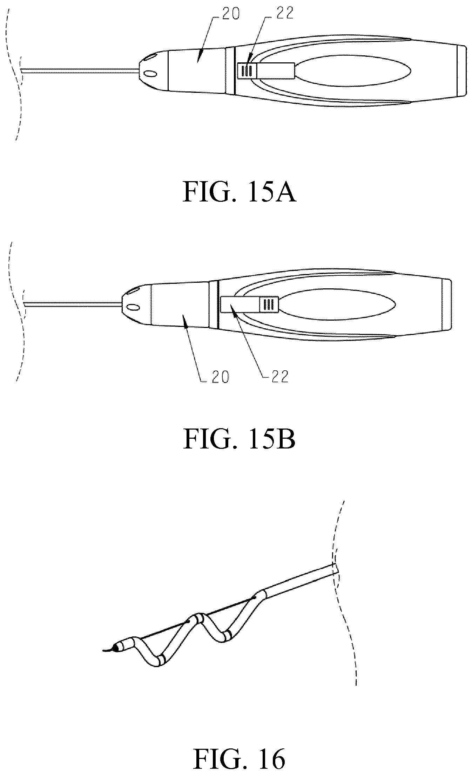

When the wall-attaching adjustment wire 6 is combined with the supporting wire 7, or the supporting wire 7 has the function of the shaping wire 8, there can be only one control element 22 connected with the wall-attaching adjustment wire 6 on the control handle 20. Please see Fig. 15A and Fig. 15B for the structure of the control handle 20 in this case. By pushing the control element 22 back to the position as shown in Fig. 15B from the position as shown in Fig. 15A, the wall-attaching adjustment wire 6 can be pulled back, so that the diameter of the electrode frame is increased.

### Sixth embodiment

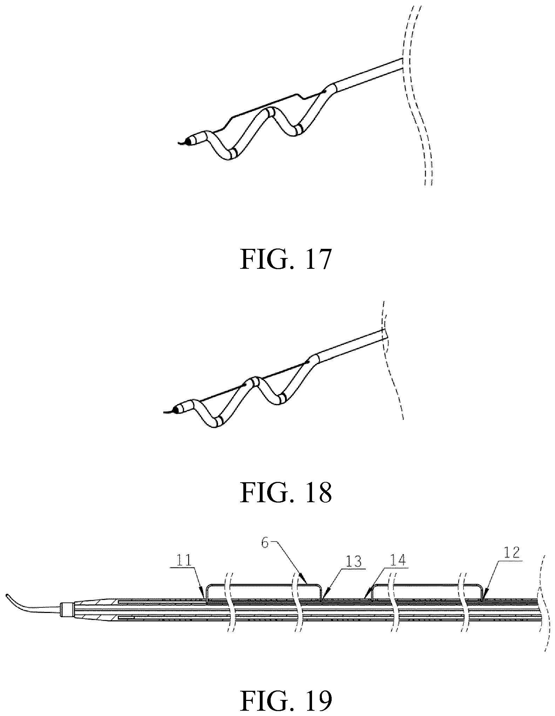

Fig. 16 and Fig. 17 are two structure diagrams of the radio frequency ablation catheter in the sixth embodiment.

From the first embodiment to the fifth embodiment, whether the multiple corrugations are distributed in the same plane or in different planes, and whether the wall-attaching adjustment wire 6 runs through the holes provided on the corrugations, the wall-attaching adjustment wire 6 is arranged near the center of the electrode frame. The sixth embodiment is different from all the five embodiments mentioned above in that the wall-attaching adjustment wire 6 in the present embodiment is eccentrically arranged on the corrugated electrode frame, and the wall-attaching adjustment wire 6 can be located at the highest point of the electrode frame and can also be located at any position between the center and the vertex of the electrode frame.

In the structure as shown in Fig. 16, the wall-attaching adjustment wire 6 is eccentrically arranged on the corrugated electrode frame, and the front section of the wall-attaching adjustment wire 6 penetrates out of the hole near the rear extremity of the electrode frame, runs through the holes provided on the corrugations, then runs through the hole near the front extremity of the electrode frame and enters the front extremity of the electrode frame to be fixed.

In the structure as shown in Fig. 17, the wall-attaching adjustment wire 6 is eccentrically arranged on the corrugated electrode frame, and the front section of the wall-attaching adjustment wire 6 penetrates out of the hole near the rear extremity of the electrode frame, runs around the multiple corrugations, runs through the hole near the front extremity of the electrode frame and then enters the front extremity of the electrode frame to be fixed.

When the front extremity of the wall-attaching adjustment wire 6 runs around the multiple corrugations, the diameter of the contracted corrugated shape can be greatly increased by pulling the wall-attaching adjustment wire 6, and ideally, the electrode frame can be adapted to blood vessels with diameters larger than the diameter of the corrugation section of the electrode frame. Due to the fact that the range of the diameters of human blood vessels is fixed, the initial diameter of the corrugation shape of the electrode frame in the radio frequency ablation catheter can be reduced substantially, so that the radio frequency ablation catheter can enter blood vessels and move in blood vessels easily.

### Seventh embodiment

In the radio frequency ablation catheter as shown in Fig. 18, the electrode frame has two corrugations, the wall-attaching adjustment wire 6 is eccentrically arranged, and the wall-attaching adjustment wire 6 can be composed of one filament or two filaments.

In the structure as shown in Fig. 19, the wall-attaching adjustment wire 6 is composed of one filament, the rear section of the wall-attaching adjustment wire 6 runs through the lumen in the connecting catheter and returns to the interior of the control handle, and the rear extremity of the wall-attaching adjustment wire 6 is fixed to the control element provided on the control handle or the control element arranged externally; the middle section of the wall-attaching adjustment wire 6 penetrates out of the hole 12 near the rear extremity of the electrode frame, and then two points are fixed to the interior of a hole 13 and a hole 14 provided at the crest at the middle position between two corrugations respectively; then the front extremity of the wall-attaching adjustment wire 6 runs through the hole 11 near the front extremity of the electrode frame, enters the interior of the electrode frame, runs through the lumens in the electrode frame and the connecting catheter, returns to the rear extremity of the connecting catheter, and is fixed to the same control element or to different control elements respectively with the rear extremity. In such a structure, both the front section and the rear section of the wall-attaching adjustment wire 6 run through the lumen inside the connecting catheter, the front extremity and the rear extremity of the wall-attaching adjustment wire 6 are fixed to the corresponding control elements respectively, and both of the two corresponding control elements can be arranged on the control handle 20 or outside the control handle 20, or one control element is arranged on the control handle 20, and the other control element is arranged outside the control handle 20. The front section and the rear section of the wall-attaching adjustment wire 6 are controlled through the two corresponding control elements respectively, and the contraction degrees of two corrugations can be separately adjusted. Furthermore, the front extremity and the rear extremity of the wall-attaching adjustment wire 6 can be fixed to the same control element.

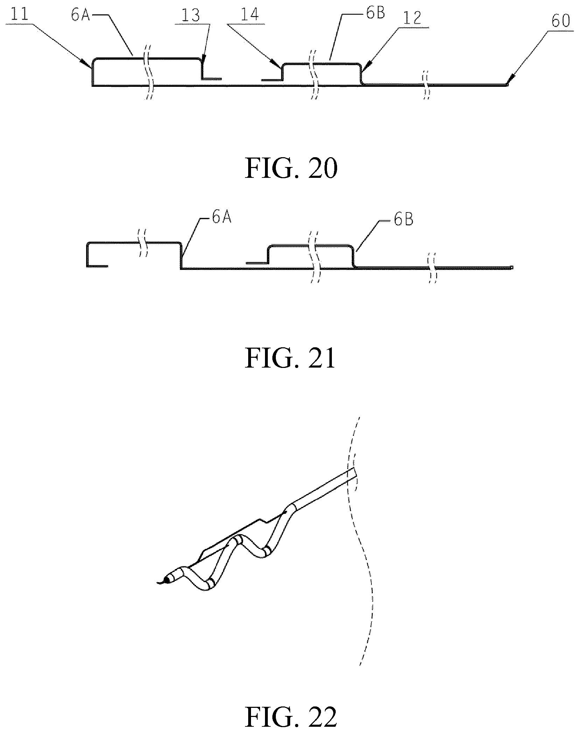

As shown in Fig. 20 and Fig. 21, in the structure as shown in Fig. 18, the wall-attaching adjustment wire 6 can also be composed of two filaments 6A and 6B used for adjusting two corrugations respectively, the front extremity of each filament is fixed to one end of the corresponding corrugation, and the other extremity of each filament runs around the corresponding corrugation, returns to the interior of the electrode frame from the other end of the corrugation, runs through the lumens in the electrode frame and the connecting catheter, returns to the control handle, and is then fixed to the corresponding control element arranged on the control handle or arranged externally.

In Fig. 20, the front extremity of the filament 6A is fixed in the hole 13 provided between the two corrugations, and the rear extremity of the filament 6A runs through the hole 11 near the front extremity of the electrode frame, returns to the interior of the electrode frame, runs through the lumens in the electrode frame and the connecting catheter, returns to the control handle, and is then fixed to the corresponding control element; the front extremity of the filament 6B is fixed in the other hole 14 provided between the two corrugations, and the rear extremity of the filament 6B runs through the hole 12 near the rear extremity of the electrode frame, returns to the interior of the electrode frame, runs through the lumens in the electrode frame and the connecting catheter, returns to the control handle, and is then fixed to the corresponding control element. In Fig. 21, the filament 6B is arranged in the same way as Fig. 20, the front extremity of the filament 6A is fixed in the hole 11 near the front extremity of the electrode frame, and the rear extremity of the filament 6A runs through the hole 13 provided between the two corrugations, returns to the interior of the electrode frame, runs through the lumens in the electrode frame and the connecting catheter, and is then fixed to the corresponding control element. The two corresponding control elements fixed to the filament 6A and the filament 6B respectively can be arranged on the control handle 20 or outside the control handle 20. The wall-attaching adjustment wire parts 6A and 6B are used for controlling the contraction degrees of the two corrugations respectively. The wall-attaching adjustment wire parts 6A and 6B are controlled through the two corresponding control elements respectively, and the contraction degrees of two corrugations can be separately adjusted. Furthermore, the corresponding control elements of the filament 6A and the filament 6B can be the same control element.

### Eighth embodiment

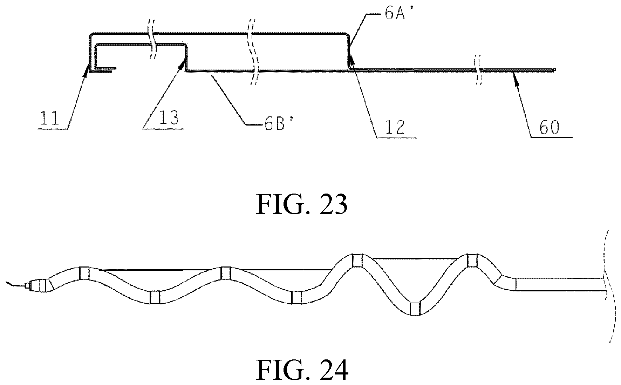

In the eighth embodiment as shown in Fig. 22 and Fig. 23, the wall-attaching adjustment wire 6 is composed of two filaments 6A' and 6B' used for adjusting one corrugation and one section of corrugations (namely a corrugation section) respectively, the front extremity of each filament is fixed to one end of the corresponding corrugation/corrugation section, and the other extremity of each filament runs around the corresponding corrugation/corrugation section, returns to the interior of the electrode frame from the other end of the corrugation/corrugation section, runs through the lumens in the electrode frame and the connecting catheter, returns to the control handle, and is fixed to the corresponding control elements. As shown in Fig. 23, the front extremity of the filament 6A' and the front extremity of the filament 6B' are both fixed in the hole 11 near the front extremity of the electrode frame, and the rear extremities 60 of the two filaments run through the hole 13 provided between two corrugations and the hole 12 near the rear extremity of the electrode frame respectively, return to the interior of the electrode frame, and are finally fixed to the corresponding control elements. The filament 6A' is used for controlling the contraction degree of the single corrugation near the front extremity of the electrode frame, the filament 6B' is used for controlling the whole corrugation section, in the present embodiment as shown in the figure, the whole corrugation section comprises two corrugations, that is to say, the filament 6B' is used for controlling the contraction degree of two corrugations. The corrugation section adjusted by the filament 6B' comprises the single corrugation adjusted by the filament 6A'. In the present embodiment, the corresponding control elements connected with the rear extremities of the two filaments respectively can also be one control element.

It can be learnt from the seventh embodiment and the eighth embodiment that when the electrode frame has two or more corrugations, the wall-attaching adjustment wire 6 can be composed of two or more filaments, the multiple filaments are used for adjusting one corrugation or one section of corrugations on the electrode frame respectively, wherein one section of corrugations comprises two or more corrugations, the front extremity of each filament is fixed to one end of the corresponding corrugation/corrugation section, and the other extremity of each filament runs around the corrugation/corrugation section, returns to the interior of the electrode frame from the other end of the corrugation/corrugation section, runs through the lumens in the electrode frame and the connecting catheter and is fixed to the corresponding control elements. When one filament is used for adjusting a single corrugation, the front extremity of the filament is fixed to one end of the corrugation, and the rear extremity of the filament runs through the hole formed in the other end of the corrugation and penetrates into the electrode frame; when one filament is used for adjusting a certain section of corrugations, the front extremity of the filament is fixed to one end of the section of corrugations, and the rear extremity of the filament runs through the hole formed in the other end of the section of corrugations and penetrates into the electrode frame. The multiple sections of corrugations controlled by the multiple filaments respectively can overlap. In the structure shown in Fig. 20 and Fig. 21, the wall-attaching adjustment wire has two filaments, and the two filaments are used for adjusting two corrugations on the electrode frame respectively; while in the structure of the eighth embodiment shown in Fig. 22 and Fig. 23, the wall-attaching adjustment wire 6 has two filaments, and the two filaments are used for controlling one corrugation and one section of corrugations on the electrode frame respectively.

When multiple control elements are adopted to control different corrugation sections of the electrode frame, after the radio frequency ablation catheter enters a target position, the corresponding corrugation sections of the electrode frame can be expanded in a segmented mode as needed, in other words, only the diameters of the corrugation sections requiring radio frequency are changed, in this way, the diameters of different corrugation sections of the electrode frame can be adjusted more flexibly, and the wall-attaching adjustment difficulty of the radio frequency ablation catheter is reduced.

Furthermore, the rear extremities of the multiple filaments can also be fixed to one control element, so that one control element can control all the filaments.

### Ninth embodiment

As shown in Fig. 24, the electrode frame of the radio frequency ablation catheter provided in the present embodiment is composed of multiple corrugations in different sizes. Every corrugation can have its own size, or part of the corrugations can have one size and others have a different size. Besides, the multiple corrugations are provided from the front extremity to the rear extremity of the electrode frame in a size increasing mode, or the multiple corrugations are provided from the front extremity to the rear extremity of the electrode frame in a size reducing mode. In this case, the wall-attaching adjustment wire 6 composed of multiple filaments is arranged in the radio frequency ablation catheter, the different filaments are used for controlling different parts of the electrode frame respectively, and the corrugation size of a corresponding area of a corrugation section can be changed by pulling different filaments, so that local wall attaching of the electrode frame can be achieved. Please refer to the sixth embodiment and the seventh embodiment for the specific arrangement mode of the wall-attaching adjustment wire 6 composed of multiple filaments, and the descriptions thereof are omitted herein.

When the multiple corrugations are provided from the front extremity to the rear extremity of the electrode frame in a size increasing mode, the radio frequency ablation catheter using the electrode frame is suitable for the situation that the diameter of a target lumen becomes smaller gradually. For example, the radio frequency ablation catheter can enter a small branch blood vessel with a small diameter from a blood vessel with a large diameter for ablation. In this case, the multiple filaments corresponding to small-diameter corrugation sections can be controlled to allow the small-diameter corrugation sections to be well attached to the wall, so that the small branch blood vessel can be ablated by means of the small-diameter corrugation sections; or, large-diameter corrugation sections and the small-diameter corrugation sections can be attached to the wall at the same time by controlling the multiple filaments, so that the large blood vessel and the small blood vessel can be ablated at the same time or in sequence.

When the multiple corrugations are provided from the front extremity to the rear extremity of the electrode frame in a size reducing mode, the radio frequency ablation catheter using the electrode frame is suitable for the situation that the diameter of a target lumen becomes larger gradually. For example, the radio frequency ablation catheter is suitable for sympathetic denervation ablation of the pelvis region via the urethral system, the catheter runs through the urethra, enters the bladder, enters the fallopian tube and reaches the pelvis region, at the moment, the large-diameter corrugation sections can be well attached to the wall of the pelvis region and the small-diameter corrugation sections can be well attached to the wall of the fallopian tube by adjusting the wall-attaching adjustment wire, so that sympathetic nerves near the fallopian tube and the pelvis region can be ablated at the same time.

In conclusion, the wall-attaching adjustment wire is arranged in the corrugated radio frequency ablation catheter, and the corrugation diameter of the electrode frame can be changed by pulling back the wall-attaching adjustment wire, so that the wall-attaching state of the electrodes can be improved, and the radio frequency ablation catheter can be adapted to blood vessels in different diameters. Furthermore, the wall-attaching adjustment wire can be of a multi-filament structure, so as to control different corrugation sections of the radio frequency ablation catheter, and reduce the difficulty of diameter adjustment.

In actual clinical treatment, the radio frequency ablation catheter and a radio frequency ablation apparatus provided by the present invention can be applied to different positions and blood vessels or tracheae with different diameters for neuroablation. For example, the radio frequency ablation catheter and the radiofrequency ablation apparatus can be applied to neuroablation in the renal artery to treat resistant hypertension, neuroablation in the arteria coeliaca to treat diabetes, trachea/bronchus vagus nerve branch ablation to treat asthma, and duodenum vagus nerve branch ablation to treat duodenal ulcers; besides, the radiofrequency ablation catheter and the radiofrequency ablation apparatus can also be used for neuroablation in other blood vessels or tracheae like pelvis and pulmonary artery. It should be noted that the radiofrequency ablation catheter provided by the present invention is not limited to the applications listed above, but can be applied to neuroablation of other portions.

Above is the introduction of the radiofrequency ablation catheter provided by the present invention, and the present invention also provides a radiofrequency ablation apparatus comprising the radiofrequency ablation catheter. Besides the radiofrequency ablation catheter, the radiofrequency ablation apparatus also comprises a radiofrequency ablation main unit connected with the radiofrequency ablation catheter. The wall-attaching adjustment wire inside the electrode frame is correspondingly connected to the control handle after running through the connecting catheter, and the shape of the electrode frame can be changed by pulling the wall-attaching adjustment wire through the control handle, so that the electrode frame can be well attached to the wall in target lumens with different diameters. Furthermore, the radiofrequency cables and the thermocouple wires in the electrode frame are connected to corresponding circuits in the radiofrequency ablation main unit respectively through the connecting catheter, so that the radio frequency ablation main unit can conduct radio frequency control and temperature monitoring on the multiple electrodes. The arrangement of the control handle and the radio frequency ablation main unit can be found in previous published patent applications of the applicant, and the descriptions of specific structures thereof are omitted herein.

Above is detailed description of the corrugated radio frequency ablation catheter having the wall-attaching adjustment wire and the apparatus thereof provided by the present invention. For those skilled in the art, any apparent modifications without deviating from the scope of the present invention will fall within the protection scope of the present invention, as defined by the appended claims.

## Claims

1. A corrugated radio frequency ablation catheter having a wall-attaching adjustment wire (6), provided with a strip-shaped connecting catheter, an electrode frame provided at the front extremity of the connecting catheter, and a control handle (20) provided at the rear extremity of the connecting catheter; **characterized in that**:
the electrode frame is a corrugated electrode frame consisting of multiple corrugations, where multiple electrodes (2) are distributed on the corrugations; each corrugation is in the shape of a fold line composed of several straight line segments, or each corrugation is composed of several curve segments, or each corrugation is composed of several curves and straight lines;
the rear section of the wall-attaching adjustment wire is slidably provided within one lumen of the connecting catheter and is connected at the rear extremity onto a control element provided on the control handle or connected onto a control element provided outside of the control handle; the front section of the wall-attaching adjustment wire protrudes to the outside of the electrode frame and either runs through one or more holes provided on the corrugations or runs around the multiple corrugations, and then the front extremity returns to the interior of the electrode frame to be fixed.

2. The corrugated radio frequency ablation catheter according to claim 1, **characterized in that**:
after returning to the interior of the electrode frame, the front extremity of the wall-attaching adjustment wire runs through lumens in the electrode frame and the connecting catheter, returns to the rear extremity of the connecting catheter, and is fixed to the control handle or the control element.

3. The corrugated radio frequency ablation catheter according to claim 2, **characterized in that**:
the corrugated radio frequency ablation catheter further comprises a supporting wire movably provided in a certain lumen of the connecting catheter and the electrode frame.

4. The corrugated radio frequency ablation catheter according to claim 3, **characterized in that**:
the corrugated radio frequency ablation catheter further comprises a shaping wire provided within the electrode frame.

5. The corrugated radio frequency ablation catheter according to claim 3, **characterized in that**:
a second control element used for being fixed to the tail end of the supporting wire is further provided on the control handle or outside the control handle.

6. The corrugated radio frequency ablation catheter according to claim 2, **characterized in that**:
the corrugated radio frequency ablation catheter further comprises a supporting wire fixedly provided in a certain lumen of the connecting catheter and the electrode frame, and the portion, inside the electrode frame, of the supporting wire is shaped into a corrugation shape, so as to form a corrugation shaping section.

7. The corrugated radio frequency ablation catheter according to claim 1, **characterized in that**:
after returning to the interior of the electrode frame, the front extremity of the wall-attaching adjustment wire protrudes out of the front extremity of the electrode fame and is fixed to the front extremity of the electrode frame or limited outside the front extremity of the electrode frame.

8. The corrugated radio frequency ablation catheter according to claim 1, **characterized in that**:
the corrugated radio frequency ablation catheter further comprises a supporting wire provided within a certain lumen of the connecting catheter and the electrode frame, and the front extremity of the wall-attaching adjustment wire is fixed to the supporting wire; or the wall-attaching adjustment wire is a filament obtained through outward branching of the supporting wire.

9. The corrugated radio frequency ablation catheter according to claim 8, **characterized in that**:
the portion, inside the electrode frame, of the supporting wire is shaped into a corrugation shape, so as to form a corrugation shaping section.

10. The corrugated radio frequency ablation catheter according to claim 1, **characterized in that**:
the corrugated radio frequency ablation catheter further comprises a shaping wire provided within a certain lumen of the electrode frame, and the front extremity of the wall-attaching adjustment wire is fixed to the shaping wire; or the wall-attaching adjustment wire is a filament obtained through outward branching of the shaping wire.

11. The corrugated radio frequency ablation catheter according to claim 1, **characterized in that**:
the wall-attaching adjustment wire is composed of two or more filaments, the multiple filaments are used for adjusting one corrugation or one section of corrugations on the electrode frame respectively, one section of corrugations comprises two or more corrugations, the front extremity of each filament is fixed to one end of the corresponding corrugation/corrugation section, and the other extremity of each filament runs around the corresponding corrugation/corrugation section, runs through lumens in the electrode frame and the connecting catheter, and is then fixed to the corresponding control element provided on the control handle or arranged externally.

12. The corrugated radio frequency ablation catheter according to claim 1, **characterized in that**:
the wall-attaching adjustment wire is eccentrically provided on the electrode frame.

13. The corrugated radio frequency ablation catheter according to claim 1, **characterized in that**:
the multiple corrugations constituting the electrode frame are provided from the front extremity to the rear extremity of the electrode frame in a size increasing mode, or the multiple corrugations constituting the electrode frame are provided from the front extremity to the rear extremity of the electrode frame in a size reducing mode.

14. The corrugated radio frequency ablation catheter according to claim 1, **characterized in that**:
a profile projections of the corrugations in the corrugated electrode frame are distributed in a crossing mode, and the multiple electrodes are arranged at the crest positions respectively.

15. A radio frequency ablation apparatus, **characterized by** comprising the radio frequency ablation catheter according to any one of claims 1-14, and a radio frequency ablation main unit connected with the radio frequency ablation catheter.

## Patentansprüche

1. Gewellter Hochfrequenz-Ablationskatheter mit einem Wandfixierungsanpassungsdraht (6), versehen mit einem streifenförmigen Anschlusskatheter, einem Elektrodenrahmen, der an dem vorderen Ende des Anschlusskatheters bereitgestellt ist, und einem Steuergriff (20), der an dem hinteren Ende des Anschlusskatheters bereitgestellt ist; **dadurch gekennzeichnet, dass**:
der Elektrodenrahmen ein gewellter Elektrodenrahmen ist, der aus mehreren Wellungen besteht, wobei mehrere Elektroden (2) an den Wellungen verteilt sind; jede Wellung die Form einer Faltlinie aufweist, die aus einigen geraden Liniensegmenten besteht, oder jede Wellung aus einigen Kurvensegmenten besteht, oder jede Wellung aus einigen Kurven und geraden Linien besteht;
die hintere Sektion des Wandfixierungsanpassungsdrahts gleitbar innerhalb eines Lumens des Anschlusskatheters bereitgestellt ist und an dem hinteren Ende auf ein Steuerelement verbunden ist, das außerhalb des Steuergriffs bereitgestellt ist; die vordere Sektion des Wandfixierungsanpassungsdrahts zu der Außenseite des Elektrodenrahmens vorsteht und entweder durch ein oder mehrere Löcher verläuft, die auf den Wellungen bereitgestellt sind, oder um die mehreren Wellungen verläuft, und dann das vordere Ende zu dem Inneren des Elektrodenrahmens zurückkehrt, um befestigt zu werden.

2. Gewellter Hochfrequenz-Ablationskatheter nach Anspruch 1, **dadurch gekennzeichnet, dass**:
nach der Rückkehr zu dem Inneren des Elektrodenrahmens, das vordere Ende des Wandfixierungsanpassungsdrahts durch Lumen in dem Elektrodenrahmen und dem Anschlusskatheter verläuft, zu dem hinteren Ende des Anschlusskatheters zurückkehrt und an dem Steuergriff oder dem Steuerelement befestigt ist.

3. Gewellter Hochfrequenz-Ablationskatheter nach Anspruch 2, **dadurch gekennzeichnet, dass**:
der gewellte Hochfrequenz-Ablationskatheter ferner einen Stützdraht umfasst, der in bestimmen Lumen des Anschlusskatheters und des Elektrodenrahmens bewegbar bereitgestellt ist.

4. Gewellter Hochfrequenz-Ablationskatheter nach Anspruch 3, **dadurch gekennzeichnet, dass**:
der gewellte Hochfrequenz-Ablationskatheter ferner einen Formdraht umfasst, der innerhalb des Elektrodenrahmens bereitgestellt ist.

5. Gewellter Hochfrequenz-Ablationskatheter nach Anspruch 3, **dadurch gekennzeichnet, dass**:
ein zweites Steuerelement, das dazu verwendet wird, um an dem hinteren Ende des Stützdrahts befestigt zu werden, ferner an dem Steuergriff oder außerhalb des Steuergriffs bereitgestellt ist.

6. Gewellter Hochfrequenz-Ablationskatheter nach Anspruch 2, **dadurch gekennzeichnet, dass**:
der gewellte Hochfrequenz-Ablationskatheter ferner einen Stützdraht umfasst, der fest in einem bestimmten Lumen des Anschlusskatheters und des Elektrodenrahmens bereitgestellt ist, und der Abschnitt, innerhalb des Elektrodenrahmens, des Stützdrahts zu einer Wellungsform geformt ist, um so eine Wellungsformsektion zu bilden.

7. Gewellter Hochfrequenz-Ablationskatheter nach Anspruch 1, **dadurch gekennzeichnet, dass**:
nach der Rückkehr zu dem Inneren des Elektrodenrahmens, das vordere Ende des Wandfixierungsanpassungsdrahts aus dem vorderen Ende des Elektrodenrahmens vorsteht und an dem vorderen Ende des Elektrodenrahmens befestigt ist oder außerhalb des vorderen Endes des Elektrodenrahmens begrenzt ist.

8. Gewellter Hochfrequenz-Ablationskatheter nach Anspruch 1, **dadurch gekennzeichnet, dass**:
der gewellte Hochfrequenz-Ablationskatheter ferner einen Stützdraht umfasst, der innerhalb eines bestimmen Lumens des Anschlusskatheters und des Elektrodenrahmens bereitgestellt ist, und das vordere Ende des Wandfixierungsanpassungsdrahts an dem Stützdraht befestigt ist; oder der Wandfixierungsanpassungsdraht ein Filament ist, das durch Auswärtsverzweigung des Stützdrahts erhalten wird.

9. Gewellter Hochfrequenz-Ablationskatheter nach Anspruch 8, **dadurch gekennzeichnet, dass**:
der Abschnitt, innerhalb des Elektrodenrahmens, des Stützdrahts zu einer Wellungsform geformt ist, um so eine Wellungsformsektion zu bilden.

10. Gewellter Hochfrequenz-Ablationskatheter nach Anspruch 1, **dadurch gekennzeichnet, dass**:
der gewellte Hochfrequenz-Ablationskatheter ferner einen Stützdraht umfasst, der innerhalb eines bestimmen Lumens des Elektrodenrahmens bereitgestellt ist, und das vordere Ende des Wandfixierungsanpassungsdrahts an dem Formdraht befestigt ist; oder der Wandfixierungsanpassungsdraht ein Filament ist, das durch Auswärtsverzweigung des Formdrahts erhalten wird.

11. Gewellter Hochfrequenz-Ablationskatheter nach Anspruch 1, **dadurch gekennzeichnet, dass**:
der Wandfixierungsanpassungsdraht aus zwei oder mehr Filamenten besteht, wobei die mehreren Filamente jeweils zum Anpassen einer Wellung oder einer Sektion von Wellungen an dem Elektrodenrahmen verwendet werden, eine Sektion von Wellungen zwei oder mehr Wellungen umfasst, das vordere Ende jedes Filaments an einem Ende der entsprechenden Wellung/Wellung-Sektion befestigt ist, und das andere Ende jedes Filaments um die entsprechende Wellung/Wellung-Sektion verläuft, durch Lumen in dem Elektrodenrahmen und dem Anschlusskatheter verläuft und dann an dem entsprechenden Steuerelement befestigt ist, das an dem Steuergriff bereitgestellt ist oder extern angeordnet ist.

12. Gewellter Hochfrequenz-Ablationskatheter nach Anspruch 1, **dadurch gekennzeichnet, dass**:
der Wandfixierungsanpassungsdraht exzentrisch an dem Elektrodenrahmen bereitgestellt ist.

13. Gewellter Hochfrequenz-Ablationskatheter nach Anspruch 1, **dadurch gekennzeichnet, dass**:
die mehreren Wellungen, die den Elektrodenrahmen bilden, von dem vorderen Ende zu dem hinteren Ende des Elektrodenrahmens in einem Modus zunehmender Größe bereitgestellt sind, oder die mehreren Wellungen, die den Elektrodenrahmen bilden, von dem vorderen Ende zu dem hinteren Ende des Elektrodenrahmens in einem Modus abnehmender Größe bereitgestellt sind.

14. Gewellter Hochfrequenz-Ablationskatheter nach Anspruch 1, **dadurch gekennzeichnet, dass**:
Profilvorsprünge der Wellungen in dem gewellten Elektrodenrahmen in einem Kreuzmodus verteilt sind, und die mehreren Elektroden jeweils an den Kammpositionen angeordnet sind.

15. Hochfrequenz-Ablationsvorrichtung, **dadurch gekennzeichnet, dass** sie den Hochfrequenz-Ablationskatheter nach einem der Ansprüche 1 bis 14 und eine Hochfrequenz-Ablationshaupteinheit umfasst, die mit dem Hochfrequenz-Ablationskatheter verbunden ist.

## Revendications

1. Cathéter d'ablation par radiofréquences ondulé comportant un câble de réglage (6) assurant la fixation à la paroi, doté d'un cathéter de connexion en forme de bande, d'un cadre d'électrodes prévu à l'extrémité avant du cathéter de connexion, et d'une poignée de commande (20) prévue à l'extrémité arrière du cathéter de connexion ; **caractérisé en ce que** :
le cadre d'électrodes est un cadre d'électrodes ondulé consistant en des ondulations multiples où des électrodes (2) multiples sont réparties sur les ondulations ; chaque ondulation à la forme d'une ligne pliée composée de plusieurs segments de ligne droite, ou bien chaque ondulation est composée de plusieurs segments de courbe, ou bien chaque ondulation est composée de plusieurs courbes et lignes droites ;
la section arrière du câble de réglage assurant la fixation à la paroi est prévue en coulissement à l'intérieur d'une lumière du cathéter de connexion et est connectée par l'extrémité arrière à un élément de commande prévu sur la poignée de commande ou connectée sur un élément de commande prévu hors de la poignée de commande ; la section avant du câble de réglage assurant la fixation à la paroi fait saillie vers l'extérieur du cadre d'électrodes et passe soit à travers un ou plusieurs trous prévus sur les ondulations ou passe autour de multiples ondulations, puis l'extrémité avant retourne vers l'intérieur du cadre d'électrodes à fixer.

2. Cathéter d'ablation par radiofréquences ondulé selon la revendication 1, **caractérisé en ce que** :
après être retournée vers l'intérieur du cadre d'électrodes, l'extrémité avant du câble de réglage assurant la fixation à la paroi passe à travers des lumières dans le cadre d'électrodes et le cathéter de connexion, retourne vers l'extrémité arrière du cathéter de connexion, et est fixée à la poignée de commande ou à l'élément de commande.

3. Cathéter d'ablation par radiofréquences ondulé selon la revendication 2, **caractérisé en ce que** :
le cathéter d'ablation par radiofréquences ondulé comprend en outre un câble de support prévu mobile dans une certaine lumière du cathéter de connexion et du cadre d'électrodes.

4. Cathéter d'ablation par radiofréquences ondulé selon la revendication 3, **caractérisé en ce que** :
le cathéter d'ablation par radiofréquences ondulé comprend en outre un câble de mise en forme prévu à l'intérieur du cadre d'électrodes.

5. Cathéter d'ablation par radiofréquences ondulé selon la revendication 3, **caractérisé en ce que** :
un second élément de commande utilisé pour être fixé à l'extrémité arrière du câble de support est en outre prévu sur la poignée de commande ou hors de la poignée de commande.

6. Cathéter d'ablation par radiofréquences ondulé selon la revendication 2, **caractérisé en ce que** :
le cathéter d'ablation par radiofréquences ondulé comprend en outre un câble de support prévu de manière fixe dans une certaine lumière du cathéter de connexion et du cadre d'électrode, et la partie, à l'intérieur du cadre d'électrodes, du câble de support est formée de façon ondulée, de façon à former une section formant une ondulation.

7. Cathéter d'ablation par radiofréquences ondulé selon la revendication 1, **caractérisé en ce que** :
après être retournée vers l'intérieur du cadre d'électrodes, l'extrémité avant du câble de réglage assurant la fixation à la paroi fait saillie hors de l'extrémité avant du cadre d'électrodes et est fixée à l'extrémité avant du cadre d'électrodes ou limitée hors de l'extrémité avant du cadre d'électrodes.

8. Cathéter d'ablation par radiofréquences ondulé selon la revendication 1, **caractérisé en ce que** :
le cathéter d'ablation par radiofréquences ondulé comprend en outre un câble de support prévu dans une certaine lumière du cathéter de connexion et du cadre d'électrodes, et l'extrémité avant du câble de réglage assurant la fixation à la paroi est fixée au câble de support ; ou bien le câble de réglage assurant la fixation à la paroi est un filament obtenu par un branchement extérieur du câble de support.

9. Cathéter d'ablation par radiofréquences ondulé selon la revendication 8, **caractérisé en ce que** :
la partie, à l'intérieur du cadre d'électrodes, du câble de support est formée de façon ondulée, de façon à former une section formant une ondulation.

10. Cathéter d'ablation par radiofréquences ondulé selon la revendication 1, **caractérisé en ce que** :
le cathéter d'ablation par radiofréquences ondulé comprend en outre un câble de conformation prévu dans une certaine lumière du cadre d'électrodes, et l'extrémité avant du câble de réglage assurant la fixation à la paroi est fixée au câble de support ; ou bien le câble de réglage assurant la fixation à la paroi est un filament obtenu par un branchement extérieur du câble de conformation.

11. Cathéter d'ablation par radiofréquences ondulé selon la revendication 1, **caractérisé en ce que** :
le câble de réglage assurant la fixation à la paroi est composé de deux filaments ou plus, les filaments multiples sont utilisés pour régler une ondulation ou une section d'ondulations du cadre d'électrodes respectivement, une section d'ondulations comprend deux ondulations ou plus, l'extrémité avant de chaque filament est fixée à une extrémité de l'ondulation/la section d'ondulations correspondante, et l'autre extrémité de chaque filament passe autour de l'ondulation/la section d'ondulations correspondante, passe à travers des lumières dans le cadre d'électrodes et le cathéter de connexion, et est ensuite fixée à l'élément de commande correspondant prévu sur la poignée de commande ou agencé de manière externe.

12. Cathéter d'ablation par radiofréquences ondulé selon la revendication 1, **caractérisé en ce que** :
le câble de réglage assurant la fixation à la paroi est prévu de manière excentrée sur le cadre d'électrodes.

13. Cathéter d'ablation par radiofréquences ondulé selon la revendication 1, **caractérisé en ce que** :
les ondulations multiples, constituant le cadre d'électrodes sont prévues de l'extrémité avant vers l'extrémité arrière du cadre d'électrodes avec une augmentation de taille, ou bien les ondulations multiples, constituant le cadre d'électrodes sont prévues de l'extrémité avant vers l'extrémité arrière du cadre d'électrodes avec une réduction de taille.

14. Cathéter d'ablation par radiofréquences ondulé selon la revendication 1, **caractérisé en ce que** :
des saillies de profilé des ondulations dans le cadre d'électrodes ondulé sont réparties en se croisant, et les électrodes multiples sont agencées sur les positions de crête respectivement.

15. Appareil d'ablation par radiofréquences, **caractérisé en ce qu'**il comprend le cathéter d'ablation par radiofréquences selon l'une quelconque des revendications 1-14, et une unité principale d'ablation par radiofréquences connectée au cathéter d'ablation par radiofréquences.
